(19) Europäisches Patentamt
European Patent Office
Office européen des brevets

(11) EP 0 815 846 B1

(12) **FASCICULE DE BREVET EUROPEEN**

(45) Date de publication et mention de la délivrance du brevet:
**25.11.1998 Bulletin 1998/48**

(51) Int. Cl.$^6$: **A61K 7/48**, A61K 7/06, A61K 7/00

(21) Numéro de dépôt: **97401256.9**

(22) Date de dépôt: **04.06.1997**

(54) **Emulsion huile-dans-eau ultrafine contenant un poly(acide 2-acrylamido-2-méthylpropane sulfonique) réticulé et neutralisé à au moins 90% et procédé de préparation**

Ultrafeine Öl-in-Wasser Emulsion, die eine vernetzte, zu mindestens 90% neutralisierte Poly(-2-Acrylamide-2-Methylpropane Sulfonsäure) enthält und Verfahren zur Herstellung

Ultrafine oil-in-water emulsion containing a crosslinked at least 90% neutralized polymer of 2-acrylamide-2-methylpropane sulfonic acid and method of preparation

(84) Etats contractants désignés:
**DE ES FR GB IT**

(30) Priorité: **28.06.1996 FR 9608111**

(43) Date de publication de la demande:
**07.01.1998 Bulletin 1998/02**

(73) Titulaire: **L'OREAL**
**75008 Paris (FR)**

(72) Inventeur: **Lorant, Raluca**
**94320 Thiais (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**DE-A- 4 010 393** **US-A- 5 114 706**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.6/3.4

## Description

La présente invention a trait à une composition cosmétique et/ou dermatologique sous forme d'émulsion huile-dans-eau ultrafine susceptible d'être obtenue par inversion de phase gélifiée et stabilisée par au moins un polymère poly(acide 2-acrylamido 2-méthylpropane sulfonique) réticulé et neutralisé à au moins 90%.

Pour diverses raisons liées en particulier à un meilleur confort d'utilisation (douceur, émollience et autres), les compositions cosmétiques ou dermatologiques actuelles se présentent le plus souvent sous la forme d'une émulsion du type huile-dans-eau (c'est à dire un support constitué d'une phase continue dispersante aqueuse et d'une phase discontinue dispersée huileuse) ou une émulsion du type eau-dans-huile (c'est à dire un support constitué d'une phase continue dispersante grasse et d'une phase discontinue dispersée aqueuse). Les émulsions huile-dans-eau sont les plus demandées dans le domaine de la cosmétique du fait qu'elles apportent sur la peau à l'application un toucher plus doux, moins gras et plus léger que les systèmes d'émulsions eau-dans-huile.

Un des paramètre fondamental influençant la stabilité des émulsions huile-dans-eau(que l'on notera H/E) est la taille des gouttelettes d'huile dispersées qui est en relation avec la tension de surface entre la phase discontinue et la phase continue. Plus la taille des gouttelettes d'huile est faible, plus la tension de surface diminue et plus la stabilité de l'émulsion augmente.

Les émulsions huile-dans-eau sont stabilisées généralement par des tensio-actifs émulsionnants du type huile-dans-eau qui grâce à leur structure amphiphiles se placent à l'interface huile/eau et stabilisent ainsi les gouttelettes dispersées. L'augmentation en agent émulsionnant permet le plus souvent d'améliorer la stabilité de l'émulsion. Leur présence à de fortes concentration conduit à des effets anti-cosmétiques tels que des touchers rêches, des touchers collants ou poisseux ainsi qu'à des problèmes d'innocuité vis à vis de la peau, des yeux et du cuir chevelu.

Pour résoudre les problèmes de stabilité des émulsions H/E classiques, on a proposé de réaliser des émulsions H/E spécifiques dites "ultrafines", et pour lesquelles la taille moyenne des globules constituant la phase grasse est comprise dans des limites bien déterminées, à savoir entre 50 et 1000 nm, lesdites émulsions ultrafines de type H/E étant elles-mêmes obtenues selon une technique d'émulsification par inversion de phase.

Cette technique est décrite par exemple dans le document DE-A-4010393.
Cette technique est, dans son principe, bien connue de l'homme de l'art et est notamment décrite dans les articles "Phase Inversion Emusification", par Th Förster et al, paru dans Cosmetics & Toiletries, vol. 106, Decembre 1991, pp 49-52, 《Application of the phase-inversion-température method to the emulsification of cosmetics》 par T. MITSUI et al, paru dans American Cosmetics and Perumery, vol 87, Decembre 1972. Son principe est ainsi le suivant : on prépare une émulsion (introduction de l'eau dans l'huile) à une température qui doit être supérieure à la température d'inversion de phase (ou TIP) du système, c'est à dire la température à laquelle l'équilibre entre les propriétés hydrophiles et lipophiles du ou des émulsionnants mis en oeuvre est atteint ; à température élevée (>TIP), l'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion.

Ces émulsions sont extrêmement fluides ; elles présentent un aspect bleuté et sont translucides. Ces types d'émulsions sont fragiles et posent encore un certain nombre de problèmes de stabilité. On observe encore des phénomènes de crémage, des déphasages après plusieurs cycles de congélation, des volutes et des dépôts difficilement identifiables. D'autre part, la réalisation des émulsions ultrafines impose l'utilisation d'huiles et d'agents émulsionnants très spécifiques dans des conditions particulières et l'ajout de certains actifs et d'ingrédients cosmétiques est délicate et conduit souvent à une déstabilisation de l'émulsion. Enfin, l'extrême fluidité de ces émulsions limite leur utilisation, dans une gamme de viscosité très restreinte, dans des domaines cosmétiques ou dermatologiques très particuliers et ne convient pas à toutes les peaux.

La gélification de ce type d'émulsion devrait en théorie permettre d'améliorer leur stabilité et d'élargir la gamme des viscosités de manière à pouvoir l'utiliser dans tous les domaines cosmétiques et dermatologiques et de diversifier ses textures afin de les adapter à tous les types de peau.

Malheureusement, les agents gélifiants couramment utilisés en cosmétique sont pour la plupart peu performants et certains sont incompatibles avec ce type d'émulsion. De manière générale, les gélifiants classiques ne permettent pas d'épaissir de façon homogène les émulsions obtenues par inversion de phase et de les stabiliser dans différentes gammes de viscosité.

En particulier, les copolymères acrylate/acide acrylique tels que le produit SALCARE SC 97 déstabilisent les émulsions obtenues par inversion de phase et les copolymères acrylamide/acide 2-acrylamido 2-méthyl propane sulfonique tels que les produits SEPIGEL conduisent à des émulsions hétérogènes et à des phénomènes de crémage. Les dérivés de cellulose et les polyacryliques du type CARBOPOL confèrent un toucher collant et n'améliorent pas les émulsions obtenues par inversion de phase posant des problèmes de stabilité.

La demanderesse a découvert de manière surprenante une nouvelle famille de polymères épaississants et/ou gélifiants permettant de réaliser des émulsions obtenues par inversion de phase, stables dans toutes les gammes de viscosité (par exemple allant de 10 à 30000 mPa.s soit 10 à 30000 cps) avec une grande variété d'émulsionnants possibles et quelles que soient les huiles utilisées. Cette nouvelle famille permet de résoudre tous les problèmes spécifiques à ce type d'émulsion qui ont été évoqués ci-dessus.

La demanderesse a découvert également que cette nouvelle famille de polymères épaississants et/ou gélifiants permettait de réaliser des émulsions ultrafines pouvant contenir une large gamme d'actifs et d'adjuvants cosmétiques sans perturber leur stabilité.

Utilisés à faible taux, les gélifiants de l'invention que l'on définiera plus loin, ne modifient pas la viscosité extrêmement fluide des émulsions obtenues par inversion de phase les contenant mais rend celles-ci filmogènes, plus douces et confortables à l'application.

En augmentant progressivement le taux de gélifiant conforme à l'invention, on peut obtenir des laits onctueux plus ou moins fluides, frais et confortables ou bien des crèmes plus ou moins épaisses, onctueuses, douces, riches et légères à la fois.

Cette diversification des textures ainsi obtenue par les gélifiants de l'invention permet de multiplier les domaines d'utilisation des émulsions obtenues par inversion de phase et de pouvoir les adapter à tous les types de peau.

Les émulsions huile/eau ultrafines de l'invention gélifiées par les polymères spécifiques que l'on définira par la suite, présentent par rapport aux émulsions huile/eau classiques, outre leur plus grande stabilité, des propriétés cosmétiques sensiblement améliorées telles que la douceur, la fraîcheur et le confort à l'application ainsi qu'une sensation de fondant et de glissant originale.

La présente invention concerne une composition cosmétique et/ou dermatologique sous forme d'émulsion huile-dans-eau ultrafine dans laquelle la taille moyenne des globules qui constituent la phase huileuse est comprise entre 50 nm et 1000 nm (nanomètres), caractérisée par le fait qu'elle contient au moins un polymère poly(acide 2-acrylamido 2-méthylpropane sulfonique) réticulé et neutralisé à au moins 90%.

Les émulsions huile-dans-eau de l'invention sont susceptibles d'êtres obtenues selon la technique d'émulsification par inversion de phase.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

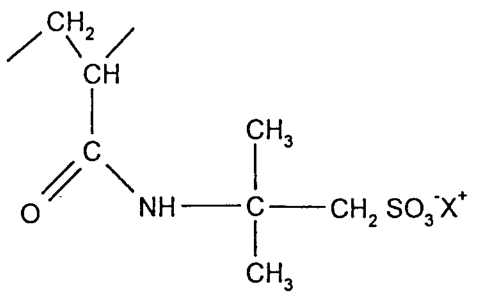

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyléther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

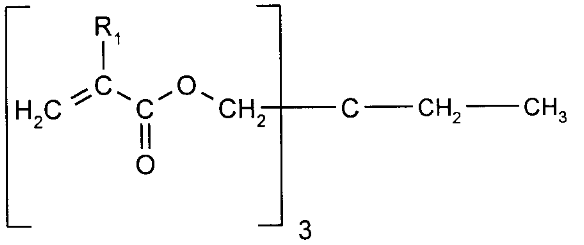
(2)

dans laquelle $R_1$ désigne un atome d'hydrogène ou un alkyle en $C_1$-$C_4$ et plus particulièrement l'hydrogène (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec :

- M désignant la masse en grammes de la macromolécule non-dissoute ;
- $N_A$ désignant le nombre d'Avogadro ;
- $V_1$ désignant le volume spécifique du solvant ;
- $V_2$ désignant le volume spécifique de la macromolécule ;
- d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al , Macromolecules, 1995, 28,4914-4919.

Les polymères particulièrement préférés sont ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 mPa.s (1000 cps) et plus préférentiellement allant de 5000 à 40000 mPa.s (5000 à 40000 cps) et plus particulièrement de 6500 à 35000 mPa.s (6500 à 35000 cps).

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer :

- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de Purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-$\alpha$-oléfines.

Comme autres huiles utilisables dans les émulsions selon l'invention, on peut encore citer les benzoates d'alcools gras en $C_{12}$-$C_{15}$ (Finsolv TN de FINETEX), les alcools gras tels que l'alcool laurique, cétylique, myristique, stéarilique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en $C_{10}$-$C_{18}$, les huiles fluorées et perfluorées, la lanoline, la lanoline hydrogénée, la lanoline acétylée et enfin les huiles de silicones, volatiles ou non comme les polyméthylsiloxanes, les polyméthylphénylpolysiloxanes, les silicones fluorées, les polysiloxanes modifiés par des alcools gras ou des polyoxyalkylènes.

Selon une caractéristique essentielle des compositions conformes à la présente invention, la taille moyenne des particules liquides (ou globules) de phase grasse au sein de la phase aqueuse dispersante doit être comprise dans des

limites bien particulières, à savoir entre 50 nm et 1000 nm. De préférence, cette taille moyenne est comprise entre 70 nm et 350 nm et plus particulièrement entre 70 et 300 nm.

De façon habituelle, les compositions selon l'invention sont caractérisées par une polydispersité très faible. En règle générale, 90% des particules de l'émulsion huile-dans-eau ultra-fine selon l'invention, ont une taille comprise entre 100 et 300 nm. La différence de taille entre les plus grandes et les plus petites particules est généralement comprise entre 20 et 400 nm et préférentiellement entre 30 et 200 nm, alors que dans les émulsions classiques ( autres qu'émulsions PIT ou microémulsions) la différence de taille entre les plus grandes et les plus petites particules est généralement supérieure à 1000 nm.

De manière classique, la phase aqueuse dispersante peut être constituée par de l'eau, ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles.

Parmi les adjuvants cosmétiques classiques susceptibles d'être contenus dans la phase aqueuse et/ou dans la phase grasse des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants, les colorants, ou tout autre ingrédient habituellement utilisé dans le domaine des produits solaires.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les émulsions conformes à l'invention contiennent en outre généralement des tensio-actifs ou émulsionnants particuliers dont l'emploi a été rendu nécessaire pour la préparation et l'obtention de l'émulsion ultrafine. Ce point sera détaillé par la suite. Elles peuvent en outre contenir des co-émulsionnants spécifiques dont le rôle est, lors de la préparation de l'émulsion, de diminuer de manière substantielle la quantité d'agents tensio-actifs nécessaire à la réalisation de l'émulsion.

Les émulsionnants utilisables dans la présente invention sont préférentiellement choisis parmi les composés non-ioniques constitués d'une part d'un reste lipophile choisi par exemple parmi les fonctions alkyle ou acyle en $C_6$-$C_{30}$ et d'autre part d'un reste hydrophile choisi par exemple parmi les groupements glycol, glucose et les éthers de polyols. Leur balance HLB peut aller de 9 à 18 et plus préférentiellement de 9,5 à 11,5

On calcule la balance HLB (balance hydrophile-lipophile) d'un émulsionnant suivant la formule suivante :

$$HLB= \frac{100-L}{5}$$

dans laquelle L représente le pourcentage en poids du groupement lipophile (soit du groupement alkyle ou acyle en $C_8$-$C_{28}$) par rapport au poids de la molécule entière.

On peut citer en particulier comme émulsionnants non-ioniques préférentiellement utilisés dans la présente invention, les produits d'addition d'oxyde d'éthylène sur des alcools gras ayant 6 à 30 atomes de carbone ou sur des esters parties de polyols ayant 3 à 16 atomes de carbone et d'acides gras ayant 14 à 22 atomes de carbone. Les produits d'addition d'oxyde d'éthylène sur des alcools gras sont disponibles dans le commerce. Les produits d'addition d'oxyde d'éthylène sur des esters partiels de polyols et d'acides gras peuvent être obtenus facilement par éthoxylation d'esters partiels d'acides gras du glycérol ou de mono ou de di-esters d'acides gras du sorbitol.

De façon préférentielle, on utilisera un émulsionnant répondant à la formule (IV) :

$$R^4\text{-}(O\text{-}CH_2\text{-}CH_2)_n\text{-}OH \qquad (IV)$$

dans laquelle $R^4$ représente un résidu hydrocarboné saturé ou insaturé, ramifié ou linéaire, ayant de 8 à 28 atomes de carbone et n représente un nombre allant de 8 à 50, préférentiellement de 8 à 30 ; on peut également utiliser un produit d'addition de 4 à 20 moles d'oxyde d'éthylène sur un ou plusieurs esters parties de glycérol. Par esters partiels de glycérol on entend par exemple les mélanges de mono, di- et tri-glycérides d'acides gras en $C_{10}$-$C_{20}$ obtenus par estérification d'une mole de glycérol par 1 ou 2 moles d'un acide gras en $C_{10}$-$C_{20}$. De façon préférentielle, on utilisera comme émulsionnant le produit de condensation de l'alcool béhénylique et de 9 moles d'oxydes d'éthylène, les alcools cétylique et/ou les alcools stéaryliques oxyéthylénés ayant de 12 à 15 moles d'oxyde d'éthylène.

En outre les émulsions selon l'invention peuvent comprendre en plus un co-émulsionnant que l'on choisira de préférence parmi les alcools gras en $C_{16}$-$C_{22}$ ou les esters partiels de polyols en $C_3$-$C_6$ avec des acides gras en $C_{14}$-$C_{22}$. Plus particulièrement, on choisit d'utiliser des esters gras de glycérol en $C_{14}$-$C_{22}$.

De façon préférentielle, les émulsions huile-dans-eau conformes à l'invention présentent les compositions suivantes :

(i) phase aqueuse : de 50 à 95 % en poids, plus préférentiellement de 70 à 90 % en poids, par rapport à l'ensemble de la formulation,
(ii) phase huileuse : de 0,5 à 50 % en poids, plus préférentiellement de 10 à 30 % en poids, par rapport à l'ensemble de la formulation,
(iii) polymère poly(acide 2-acrylamido 2-méthylpropane sulfonique) réticulés et pratiquement ou totalement neutralisés : de 0,1 à 10% en poids, plus préférentiellement de 0,1 à 5% en poids, par rapport à l'ensemble de la formulation,
(iv) (co)émulsionnant(s) : de 0,5 à 30 % en poids, plus préférentiellement de 2 à 10% en poids, par rapport à l'ensemble de la formulation,

L'invention a également pour objet un procédé de préparation de ces émulsions. Les émulsions selon l'invention peuvent être obtenues par un procédé d'inversion de phase caractérisé en ce que l'on mélange

(A) au moins une huile cosmétique,
(B) au moins un émulsionnant et éventuellement un co-émulsionnant,
(D) de l'eau,
et éventuellement
(E) des adjuvants stables à la température d'inversion de phase,

afin d'obtenir une émulsion classique puis que l'on chauffe cette émulsion à une température située à l'intérieur ou au dessus du domaine d'inversion de phase ou que l'on prépare l'émulsion à une telle température, puis que dans un second temps on refroidit l'émulsion à une température inférieure au domaine d'inversion de phase ; on gélifie ensuite l'émulsion obtenue en ajoutant (C) un polymère poly(acide 2-acrylamido 2-méthylpropane sulfonique) réticulé et pratiquement ou totalement neutralisé tel que défini ci-dessus.

Le domaine de température d'inversion de phase est établi pour une composition donnée en mesurant la conductibilité d'un échantillon de la composition que l'on chauffe. Lorsque l'on atteint le domaine d'inversion de phase la conductibilité de l'émulsion augmente de façon très rapide. : dans le domaine d'inversion de phase on peut observer une augmentation de la conductibilité d'environ 50 microsiemens par centimètre sur un intervalle de température de 5 à 15°C, tandis qu'elle ne sera que d'environ 5 microsiemens par centimètre sur un intervalle de température équivalent en dehors du domaine d'inversion de phase.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin de la peau, du cuir chevelu ou des muqueuses ou pour le nettoyage de la peau.

Les compositions selon l'invention peuvent être utilisées comme produits capillaires.

Les compositions de l'invention peuvent être également utilisées comme produit antisolaire.

Les compositions peuvent être des produits pour le maquillage.

Un autre objet de l'invention est un procédé de traitement non-thérapeutique cosmétique de la peau, du cuir chevelu, des cheveux. des cils, des sourcils, des ongles ou des muqueuses, caractérisé par le fait qu'on applique sur le support une composition telle que définie ci-dessus, selon la technique d'utilisation habituelle de cette composition. Par exemple : application de crèmes, de laits sur la peau, le cuir chevelu et/ou les muqueuses. Le type de traitement est fonction du ou des actifs présents dans la composition.

L'invention a encore pour objet une utilisation de la composition ci-dessus pour préparer une pommade ou un onguent destiné à traiter thérapeutiquement le visage et/ou le corps humain, y compris les mains notamment pour traiter l'acné, les comédons chez les peaux grasses.

L'invention a encore pour objet une utilisation d'un polymère tel que défini ci-dessus, pour gélifier et/ou épaissir une composition cosmétique et/ou dermatologique sous forme d'émulsion huile-dans-eau dans laquelle la taille moyenne des globules qui constituent la phase huileuse est comprise entre 70 nm et 1000 nm.

L'invention a encore pour objet une utilisation d'un polymère tel que défini ci-dessus, pour stabiliser une composition cosmétique et/ou dermatologique sous forme d'émulsion huile-dans-eau dans laquelle la taille moyenne des globules qui constituent la phase huileuse est comprise entre 70 nm et 1000 nm.

Les exemples suivants illustrent l'invention sans présenter un caractère limitatif.

**EXEMPLE DE PREPARATION A**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 15000 à 35000 mPa.s (15000 cps à 35000 cps). La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

## EXEMPLE DE PREPARATION B

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 Pa.s (7000 cps).

Le rayon hydrodynamique du polymère obtenu dans une solution aqueuse déterminé par diffusion de la lumière dynamique est de 160 nm.

## EXEMPLE 1

| *Phase A* | |
|---|---|
| - Palmitate d'octyle | 5 % en poids |
| - Cyclo diméthylsiloxane | 5 % en poids |
| - Isoparaffine hydrogénée | 5 % en poids |
| - Alcool béhénique oxyéthyléné (9 OE) | 4,5 % en poids |

| *Phase B* | |
|---|---|
| - Glycérine | 3 % en poids |
| - Eau distillée | 13 % en poids |
| - Conservateur | qs |

| *Phase C* | |
|---|---|
| - Eau distillée | 50 % en poids |

| *Phase D* | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16000 Pa.s (16000 cps) dans une solution d'eau à 2% et à 25°C | 0,2-0,5-1% en poids |

On obtient selon la concentration du gélifiant (0,2 ; 0,5 ou 1% en poids), des formulations plus ou moins translucides et plus ou moins épaisses, stables après 2 mois de conservation à température ambiante, à 4°C, à 37 °C et après 1 mois à 45 °C ; stables après 10 cycles de congélation/décongélation.

**MODE OPERATOIRE**

On chauffe à 60 °C et on homogénéise les phases A et B. Sous agitation lente, on verse lentement la phase B sur la phase A et on chauffe le mélange jusqu'à la température d'inversion de phase (TIP) qui est autour de 80 °C. L'émulsion eau-dans-huile obtenue devient quasi-transparente et très bleutée. On arrête le chauffage et on verse la phase C en gardant une agitation lente. A température ambiante, on obtient une émulsion huile-dans-eau par inversion de phase que l'on verse ensuite sur la phase D et on homogénéise le tout avec une agitation appropriée comme une turbine MORITZ.

**EXEMPLES COMPARATIFS**

On compare 3 compositions de l'invention 1a, 1b, 1c selon l'exemple 1 défini ci-dessus contenant respectivement 0,2, 0,5 et 1% en poids de poly(acide 2-acrylamido 2-méthylpropane sulfonique) réticulé et neutralisé à plus de 90% à des compositions 2a, 2b, 2c et à des compositions 3a, 3b et 3c selon l'art antérieur contenant à la place du poly(acide 2-acrylamido 2-méthylpropane sulfonique) un gélifiant classique dans les mêmes concentrations que celles des compositions 1a, 1b, 1c.

Les compositions 2a, 2b, 2c et les compositions 3a, 3b et 3c ont comme formulation :

**Compositions 2a, 2b, 2c :** identiques respectivement aux compositions 1a, 1b, 1c selon l'exemple 1 mais contenant dans la phase D respectivement 0,2, 0,5 et 1% en poids d'un copolymère acrylamide/ acide 2-acrylamido 2-méthylpropane sulfonique réticulé et partiellement ou totalement neutralisé en émulsion tel que le produit SEPIGEL vendu par la société SEPPIC.

**Compositions 3a, 3b et 3c :** identiques respectivement aux compositions 1a, 1b, 1c selon l'exemple 1 mais contenant dans la phase D 0,5 % en poids d'un copolymère acide acrylique/acrylate de sodium en émulsion tel que le produit commercial SALCARE SC 97 vendu par la société ALLIED COLLOIDS.

On compare également toutes ces compositions à une composition 4 témoin identique à l'exemple 1 et ne contenant pas de phase D gélifiante.

Les compositions 1a, 1b, 1c ; 2a, 2b, 2c ; 3a, 3b, 3c et 4 sont préparées selon le mode opératoire décrit dans l'exemple 1. On observe leur aspect final et on étudie leur stabilité à la conservation ainsi qu'après 10 cycles de congélation/décongélation.

Les résultats de ces tests sont résumés dans le tableau suivant :

| COMPOSITIONS | ASPECT | STABILITE |
|---|---|---|
| 1a | bleuté, très fluide, homogène | stable après 2 mois à température ambiante, 4°C, 37°C<br>stable après 1 mois à 45 °C<br>stable après 10 cycles de congélation/décongélation |
| 1b | légèrement bleuté, onctueux, assez épais, homogène | stable après 2 mois à température ambiante, 4°C, 37°C et à 45 °C<br>stable après 10 cycles de congélation/décongélation |
| 1c | crème fine, blanche et épaisse, homogène | stable après 2 mois à température ambiante, 4°C, 37°C et à 45 °C<br>stable après 10 cycles de congélation/décongélation |
| 2a, 2b, 2c | opaques, hétérogènes | quelques heures après la fabrication, on observe un phénomène de crémage<br>2 jours après, les émulsions sont hétérogènes |
| 3a, 3b, 3c | opaques, hétérogènes | émulsions instables séparation en deux phases |
| 4 | bleuté, très fluide, homogène | quelques jours après la fabrication, apparition d'une phase gélifiée déposée au fond du flacon |

## Revendications

1. Composition cosmétique et/ou dermatologique sous forme d'émulsion huile-dans-eau dans laquelle la taille moyenne des globules qui constituent la phase huileuse est comprise entre 50 nm et 1000 nm (nanomètres), caractérisée par le fait qu'elle contient au moins un polymère poly(acide 2-acrylamido 2-méthylpropane sulfonique) réticulé et neutralisé à au moins 90%, comprenant distribués de façon aléatoire :

   a) de 90 à 99,9% en poids de motifs de formule générale (1) suivante :

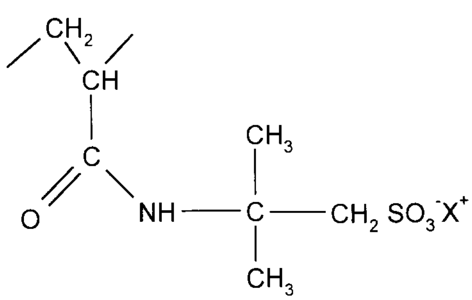

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

2. Composition selon la revendication 1, caractérisée par le fait qu'elle est susceptible d'êtres obtenue selon la technique d'émulsification par inversion de phase.

3. Composition selon la revendication 1 ou 2, caractérisée par le fait que les poly(acide 2-acrylamido 2-méthylpropane sulfonique) réticulés et neutralisés à au moins 90% comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

4. Composition selon l'une quelconque des revendications 1 à 3, caractérisée par le fait que les poly(acide 2-acrylamido 2-méthylpropane sulfonique) réticulés et neutralisés à au moins 90% comportent de 98 à 99,5 % en poids de motifs de formule (1) et de 0,2 à 2 % en poids de motifs réticulants.

5. Composition selon l'une quelconque des revendications 1 à 4, caractérisée par le fait que dans la formule (1) le cation $X^+$ est $NH_4^+$.

6. Composition selon l'une quelconque des revendications 1 à 5, caractérisée par le fait que les monomères réticulants répondent à la formule générale (2) suivante :

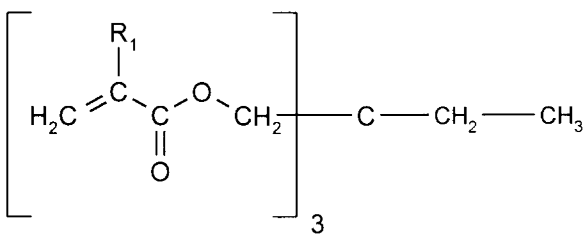

(2)

dans laquelle $R_1$ désigne hydrogène ou un alkyle en $C_1$-$C_4$.

7. Composition selon l'une quelconque des revendications 1 à 6, caractérisée par le fait que le poly(acide 2-acrylamido 2-méthylpropane sulfonique) est réticulé par le triméthylol propane triacrylate.

8. Composition selon l'une quelconque des revendications 1 à 7, caractérisée par le fait que les polymères de formule (1) présentent une viscosité mesurée au viscosimètre BROOKFIELD, mobile 4, vitesse 100 tours/minute, dans une solution d'eau à 2 % et à 25 °C, supérieure ou égale à 1000 mPa.s (1000 cps).

9. Composition selon l'une quelconque des revendications 1 à 8, caractérisée les polymères de formule (1) présentent une viscosité mesurée au viscosimètre BROOKFIELD, mobile 4, vitesse 100 tours/minute, dans une solution d'eau à 2% et à 25 °C allant de 5000 à 40000 mPa.s (5000 à 40000 cps) et plus particulièrement de 6500 à 35000 mPa.s (6500 à 35000 cps).

10. Composition selon l'une quelconque des revendications 1 à 9, caractérisée par le fait que le poly(acide 2-acrylamido 2-méthylpropane sulfonique) réticulé est présent dans dans des concentrations allant préférentiellement de 0,1 à 10% en poids par rapport au poids total de la composition et plus préférentiellement de 0,1 à 0,5% en poids.

11. Composition selon l'une quelconque des revendications 1 à 10, caractérisée par le fait que la taille moyenne des particules liquides (ou globules) de phase grasse au sein de la phase aqueuse dispersante est comprise entre 70 nm et 350 nm et de préférence entre 70 et 300 nm.

12. Composition selon l'une quelconque des revendications 1 à 11, caractérisée par le fait que 90% des particules de

l'émulsion huile-dans-eau ultra-fine ont une taille comprise entre 100 et 300 nm et que la différence de taille entre les plus grandes et les plus petites particules est comprise entre 20 et 400 nm et préférentiellement entre 30 et 200 nm.

**13.** Composition selon l'une quelconque des revendications 1 à 12, caractérisée par le fait que la phase aqueuse dispersante est constituée par de l'eau, ou un mélange d'eau et d'alcool(s) polyhydrique(s) ou bien encore un mélange d'eau et d'alcool(s) inférieur(s) hydrosoluble(s).

**14.** Composition selon l'une quelconque des revendications 1 à 13, caractérisée par le fait que la phase aqueuse dispersante représente 50 à 95 % en poids, plus préférentiellement de 70 à 90 % en poids, de la quantité totale de la composition.

**15.** Composition selon l'une quelconque des revendications 1 à 14, caractérisée par le fait que la phase grasse est constituée d'un composé ou d'un mélange de composés choisis parmi les différents corps gras, les huiles d'origine végétale, animale, synthétique ou minérale, les cires naturelles ou synthétiques, et analogues, habituellement utilisés dans les émulsions huile-dans-eau.

**16.** Composition selon l'une quelconque des revendications 1 à 15, caractérisée par le fait que la phase grasse dispersée représente 0,5 à 50 % en poids, plus préférentiellement 10 à 30 % en poids de la quantité totale de la composition.

**17.** Composition selon l'une quelconque des revendications 1 à 16, caractérisée par le fait qu'elle contient au moins un agent émulsionnant choisi parmi les composés non-ioniques constitués d'une part d'un reste lipophile choisi parmi les fonctions alkyle ou acyle en $C_6$-$C_{30}$ et d'autre part d'un reste hydrophile choisi par parmi les groupements glycol, glucose et les éthers de polyols.

**18.** Composition selon la revendication 17, caractérisée par le fait que l'agent émulsionnant présente une balance HLB (balance hydrophile-lipophile) allant de 9 à 18.

**19.** Composition selon la revendication 17 ou 18 , caractérisée par le fait que l'agent émulsionnant est choisi dans le groupe constitué par les produits d'addition d'oxyde d'éthylène sur des alcools gras ayant 6 à 30 atomes de carbone ou sur des esters partiels de polyols ayant 3 à 16 atomes de carbone et d'acides gras ayant 14 à 22 atomes de carbone.

**20.** Composition selon la revendication 19, caractérisée par le fait que l'agent émulsionnant est choisi dans le groupe constitué par

- les composés répondant à la formule (IV) :

$$R^4\text{-}(O\text{-}CH_2\text{-}CH_2)_n\text{-}OH \qquad (IV)$$

  dans laquelle $R^4$ représente un résidu hydrocarboné saturé ou insaturé, ramifié ou linéaire, ayant de 8 à 28 atomes de carbone et n représente un nombre allant de 8 à 50, préférentiellement de 8 à 30 ;
- les produits d'addition de 4 à 20 moles d'oxyde d'éthylène sur un ou plusieurs esters parties de glycérol.

**21.** Composition selon l'une quelconque des revendications 17 à 20, caractérisée par le fait qu'elle contient en outre au moins un agent co-émulsionnant choisi dans le groupe constitué par les alcools gras en $C_{16}$-$C_{22}$ ou les esters partiels de polyols en $C_3$-$C_6$ avec des acides gras en $C_{14}$-$C_{22}$.

**22.** Composition selon la revendication 21, caractérisée par le fait qu'elle contient le ou les (co)émulsionnant(s) dans des proportions allant de 0,5 à 30 % en poids, plus préférentiellement de 2 à 10% en poids, par rapport au poids total de la composition.

**23.** Composition selon l'une quelconque des revendications 1 à 22, caractérisée par le fait qu'elle contient en plus au moins un additif choisi dans le groupe constitué par les gélifiants et/ou épaississants classiques aqueux ou lipophiles ; des actifs hydrophiles ou lipophiles ; des conservateurs ;des antioxydants ; des parfums ; des agents hydratants ; des émollients ; des sequestrants ; des tensio-actifs ; des polymères ; des agents alcanisants ou acidifiants ; des charges ; des agents anti-radicaux libres ; des céramides ; des filtres solaires ; des répulsifs pour insectes;

des agents amincissants ; des matières colorantes ; des bactéricides ; des antipelliculaires.

**24.** Utilisation non-thérapeutique de la composition selon l'une quelconque des revendications 1 à 23, comme produit capillaire.

**25.** Utilisation non-thérapeutique de la composition selon l'une quelconque des revendications 1 à 23, comme produit de soin pour la peau, les cheveux, le cuir chevelu, des cils, des sourcils, des ongles ou des muqueuses .

**26.** Utilisation non-thérapeutique de la composition selon l'une quelconque des revendications 1 à 23, comme produit de maquillage.

**27.** Utilisation non-thérapeutique de la composition selon l'une quelconque des revendications 1 à 23, comme produit antisolaire.

**28.** Procédé de traitement non-thérapeutique cosmétique de la peau, du cuir chevelu, des cheveux, des cils, des sourcils, des ongles ou des muqueuses, caractérisé par le fait qu'on applique sur le support une composition telle que définie selon l'une quelconque des revendications 1 à 23.

**29.** Procédé de préparation d'une composition selon l'une quelconque des revendications 1 à 23, caractérisé par le fait qu'elle est obtenue par mélange d'au moins :

(A) au moins une huile cosmétique,
(B) au moins un émulsionnant et éventuellement un co-émulsionnant,
(D) de l'eau,
et éventuellement
(E) des adjuvants stables à la température d'inversion de phase,

afin d'obtenir une émulsion classique puis que l'on chauffe cette émulsion à une température située à l'intérieur ou au dessus du domaine d'inversion de phase ou que l'on prépare l'émulsion à une telle température, puis que dans un second temps on refroidit l'émulsion à une température inférieure au domaine d'inversion de phase ; on gélifie ensuite l'émulsion obtenue en ajoutant (C) un polymère poly(acide 2-acrylamido 2-méthylpropane sulfonique) réticulé et pratiquement ou totalement neutralisé tel que défini dans les revendications précédentes.

**30.** Utilisation de la composition selon l'une quelconque des revendications 1 à 23 pour préparer une pommade ou un onguent destiné à traiter thérapeutiquement le visage et/ou les mains et/ou le corps humain.

**31.** Utilisation d'un polymère tel que défini selon l'une quelconque des revendications 1 à 9, pour gélifier et/ou épaissir une composition cosmétique et/ou dermatologique sous forme d'émulsion huile-dans-eau ultafine dans laquelle la taille moyenne des globules qui constituent la phase huileuse est comprise entre 50 nm et 1000 nm.

**32.** Utilisation d'un polymère tel que défini selon l'une quelconque des revendications 1 à 9, pour stabiliser une composition cosmétique et/ou dermatologique sous forme d'émulsion huile-dans-eau ultrafine dans laquelle la taille moyenne des globules qui constituent la phase huileuse est comprise entre 50 nm et 1000 nm.

## Claims

**1.** Cosmetic and/or dermatological composition in the form of oil-in-water emulsion in which the mean size of the globules which form the oily phase is between 50 nm and 1000 nm (nanometres), characterized in that it contains at least one crosslinked and at least 90 % neutralized poly(2-acrylamido-2-methylpropanesulphonic acid) polymer, including, distributed randomly:

a) from 90 to 99.9 % by weight of units of following general formula (1):

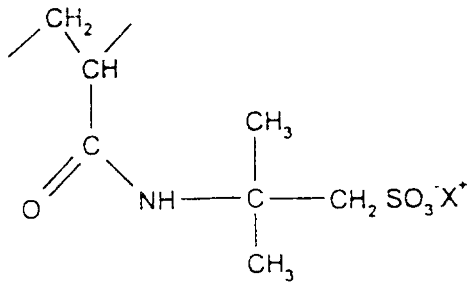

(1)

in which $X^+$ denotes a cation or a mixture of cations, it being possible for at the most 10 mol% of the cations $X^+$ to be protons $H^+$;

b) from 0.01 to 10 % by weight of crosslinking units originating from at least one monomer containing at least two olefinic double bonds, the proportions by weight being defined in relation to the total weight of the polymer.

2. Composition according to Claim 1, characterized in that it is capable of being obtained according to the emulsifying technique using phase inversion.

3. Composition according to Claim 1 or 2, characterized in that the crosslinked and at least 90 % neutralized poly(2-acrylamido-2-methylpropanesulphonic acid)s comprise a number of units of formula (1) in a quantity which is sufficiently high to obtain polymer particles whose hydrodynamic volume in solution in water has a radius ranging from 10 to 500 nm and whose distribution is homogeneous and unimodal.

4. Composition according to any one of Claims 1 to 3, characterized in that the crosslinked and at least 90 % neutralized poly(2-acrylamido-2-methylpropanesulphonic acid)s comprise from 98 to 99.5 % by weight of units of formula (1) and from 0.2 to 2 % by weight of crosslinking units.

5. Composition according to any one of Claims 1 to 4, characterized in that in formula (1) the cation $X^+$ is $NH_4^+$.

6. Composition according to any one of Claims 1 to 5, characterized in that the crosslinking monomers correspond to the following general formula (2):

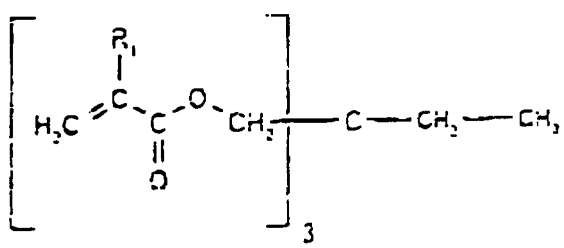

(2)

in which $R_1$ denotes hydrogen or a $C_1$-$C_4$ alkyl.

7. Composition according to any one of Claims 1 to 6, characterized in that the poly(2-acrylamido-2-methylpropanesulphonic acid) is crosslinked with trimethylolpropane triacylate.

8. Composition according to any one of Claims 1 to 7, characterized in that the polymers of formula (1) exhibit a viscosity, measured with a Brookfield viscometer, spindle 4, speed 100 revolutions/minute, in a water solution at a concentration of 2 % and at 25°C, which is higher than or equal to 1000 mPa.s (1000 cP).

9. Composition according to any one of Claims 1 to 8, characterized in that the polymers of formula (1) exhibit a viscosity, measured with a Brookfield viscometer, spindle 4, speed 100 revolutions/minute, in a water solution at a concentration of 2 % and at 25°C, ranging from 5000 to 40000 mPa.s (5000 to 40000 cP) and more particularly from 6500 to 35000 mPa.s (6500 to 35000 cP).

**10.** Composition according to any one of Claims 1 to 9, characterized in that the crosslinked poly(2-acrylamido-2-methanepropanesulphonic acid) is present in concentrations ranging preferably from 0.1 to 10 % by weight relative to the total weight of the composition, and more preferably from 0.1 to 0.5 % by weight.

**11.** Composition according to any one of Claims 1 to 10, characterized in that the mean size of the liquid particles (or globules) of fatty phase within the dispersing aqueous phase is between 70 nm and 350 nm and preferably between 70 and 300 nm.

**12.** Composition according to any one of Claims 1 to 11, characterized in that 90 % of the particles of the ultrafine oil-in-water emulsion have a size of between 100 and 300 nm and that the difference in size between the largest and the smallest particles is between 20 and 400 nm and preferably between 30 and 200 nm.

**13.** Composition according to any one of Claims 1 to 12, characterized in that the dispersing aqueous phase consists of water or a mixture of water and polyhydric alcohol(s) or else a mixture of water and of water-soluble lower alcohol(s).

**14.** Composition according to any one of Claims 1 to 13, characterized in that the dispersing aqueous phase represents 50 to 95 % by weight, more preferably from 70 to 90 % by weight, of the total quantity of the composition.

**15.** Composition according to any one of Claims 1 to 14, characterized in that the fatty phase consists of a compound or of a mixture of compounds chosen from various fatty substances, oils of vegetable, animal, synthetic or mineral origin, natural or synthetic waxes, and the like, usually employed in oil-in-water emulsions.

**16.** Composition according to any one of Claims 1 to 15, characterized in that the dispersed fatty phase represents 0.5 to 50 % by weight, more preferably 10 to 30 % by weight of the total quantity of the composition.

**17.** Composition according to any one of Claims 1 to 16, characterized in that it contains at least one emulsifying agent chosen from nonionic compounds consisting, on the one hand, of a lipophilic residue chosen from $C_6$-$C_{30}$ alkyl or acyl functional groups and, on the other hand, of a hydrophilic residue chosen from glycol groups, glucose and polyol ethers.

**18.** Composition according to Claim 17, characterized in that the emulsifying agent has an HLB (hydrophilic-lipophilic balance) ranging from 9 to 18.

**19.** Composition according to Claim 17 or 18, characterized in that the emulsifying agent is chosen from the group consisting of the products of addition of ethylene oxide to fatty alcohols containing 6 to 30 carbon atoms or to partial esters of polyols containing 3 to 16 carbon atoms and of fatty acids containing 14 to 22 carbon atoms.

**20.** Composition according to Claim 19, characterized in that the emulsifying agent is chosen from the group consisting of

- the compounds corresponding to the formula (IV):

$$R^4\text{-}(O\text{-}CH_2\text{-}CH_2)_n\text{-}OH \qquad (IV)$$

  in which $R^4$ denotes a branched or linear, saturated or unsaturated hydrocarbon residue containing from 8 to 28 carbon atoms and n denotes a number ranging from 8 to 50, preferably from 8 to 30;
- the products of addition of 4 to 20 moles of ethylene oxide to one or several partial esters of glycerol.

**21.** Composition according to any one of Claims 17 to 20, characterized in that it additionally contains at least one coemulsifying agent chosen from the group consisting of $C_{16}$-$C_{22}$ fatty alcohols or the partial esters of $C_3$-$C_6$ polyols with $C_{14}$-$C_{22}$ fatty acids.

**22.** Composition according to Claim 21, characterized in that it contains the (co)emulsifier(s) in proportions ranging from 0.5 to 30 % by weight, more preferably from 2 to 10 % by weight, relative to the total weight of the composition.

**23.** Composition according to any one of Claims 1 to 22, characterized in that it additionally contains at least one additive chosen from the group consisting of aqueous or lipophilic conventional gelling agents and/or thickeners,

hydrophilic or lipophilic active substances, preserving agents, antioxidants, perfumes, hydrating agents, emollients, sequestrants, surfactants, polymers, alkalifying or acidifying agents, fillers, agents against free radicals, ceramides, sunscreens, insect repellents, slimming agents, colouring agents, bactericides and agents against dandruff.

**24.** Nontherapeutic use of the composition according to any one of Claims 1 to 23, as a hair-care product.

**25.** Nontherapeutic use of the composition according to any one of Claims 1 to 23, as a care product for the skin, the hair, the scalp, the eyelashes, the eyebrows, the nails or the mucosae.

**26.** Nontherapeutic use of the composition according to any one of Claims 1 to 23, as a product for make-up.

**27.** Nontherapeutic use of the composition according to any one of Claims 1 to 23, as an antisun product.

**28.** Process for nontherapeutic cosmetic treatment of the skin, of the scalp, of the hair, of the eyelashes, of the eyebrows, of the nails or of the mucosae, characterized in that a composition as defined according to any one of Claims 1 to 23 is applied to the substrate.

**29.** Process for the preparation of a composition according to any one of Claims 1 to 23, characterized in that it is obtained by mixing of at least:

(A) at least one cosmetic oil,
(B) at least one emulsifier and optionally a coemulsifier,
(D) water,
and optionally
(E) adjuvants which are stable at the phase inversion temperature,

in order to obtain a conventional emulsion and in that this emulsion is then heated to a temperature situated within or above the phase inversion region or that the emulsion is prepared at such a temperature and in that in a second step the emulsion is then cooled to a temperature lower than the phase inversion region; the emulsion obtained is next gelled by adding (C) a crosslinked and practically or completely neutralized poly(2-acrylamido-2-methylpropanesulphonic acid) polymer as defined in the preceding claims.

**30.** Use of the composition according to any one of Claims 1 to 23 for preparing a salve or an ointment intended for therapeutically treating the face and/or the hands and/or the human body.

**31.** Use of a polymer as defined according to any one of Claims 1 to 9, for gelling and/or thickening a cosmetic and/or dermatological composition in the form of ultrafine oil-in-water emulsion in which the mean size of the globules which form the oily phase is between 50 nm and 1000 nm.

**32.** Use of a polymer as defined according to any one of Claims 1 to 9, for stabilizing a cosmetic and/or dermatological composition in the form of ultrafine oil-in-water emulsion in which the mean size of the globules which form the oily phase is between 50 nm and 1000 nm.

**Patentansprüche**

**1.** Kosmetische und/oder dermatologische Zusammensetzung in Form einer Öl-in-Wasser-Emulsion, in der die mittlere Größe der Kügelchen, die die Ölphase bilden, 50 bis 1000 nm (Nanometer) beträgt,
dadurch gekennzeichnet, daß
sie mindestens ein vernetztes und zu mindestens 90 % neutralisiertes Poly(2-acrylamido-2-methylpropansulfonsäure)-Polymer enthält, das zufällig verteilt enthält:

a) 90 bis 99,9 Gew.-% Einheiten der folgenden allgemeinen Formel (1)

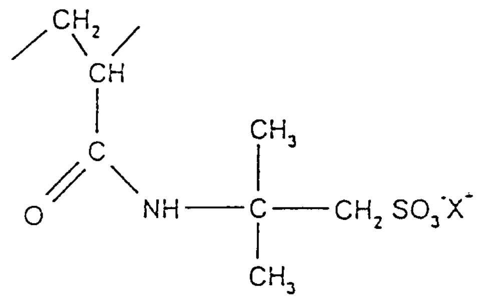

(1)

in der $X^+$ ein Kation oder ein Kationengemisch bedeutet, wobei höchstens 10 Mol-% der Kationen $X^+$ Protonen $H^+$ sein können,

b) 0,01 bis 10 Gew.-% vernetzende Einheiten, die von mindestens einem Monomer stammen, das mindestens zwei olefinische Doppelbindungen aufweist, wobei die Gewichtsanteile bezogen auf das Gesamtgewicht des Polymers angegeben sind.

2. Zusammensetzung nach Anspruch 1, dadurch gekennzeichnet, daß sie unter Anwendung der Phaseninversionsmethode erhalten wird.

3. Zusammensetzung nach Anspruch 1 oder 2, dadurch gekennzeichnet, daß die vernetzten und zu mindestens 90 % neutralisierten Poly(2-acrylamido-2-methylpropansulfonsäuren) Einheiten der Formel (1) in einer Menge enthalten, die ausreicht, um Polymerpartikel zu erhalten, deren hydrodynamisches Volumen in wäßriger Lösung einen Radius von 10 bis 500 nm aufweist und deren Verteilung homogen und unimodal ist.

4. Zusammensetzung nach einem der Ansprüche 1 bis 3, dadurch gekennzeichnet, daß die vernetzten und zu mindestens 90 % neutralisierten Poly(2-acrylamido-2-methylpropansulfonsäuren) 98 bis 99,5 Gew.-% Einheiten der Formel (1) und 0,2 bis 2 Gew.-% vernetzende Einheiten enthalten.

5. Zusammensetzung nach einem der Ansprüche 1 bis 4, dadurch gekennzeichnet, daß in der Formel (1) das Kation $X^+$ $NH_4^+$ ist.

6. Zusammensetzung nach einem der Ansprüche 1 bis 5, dadurch gekennzeichnet, daß die vernetzenden Monomere die folgende allgemeine Formel (2) aufweisen

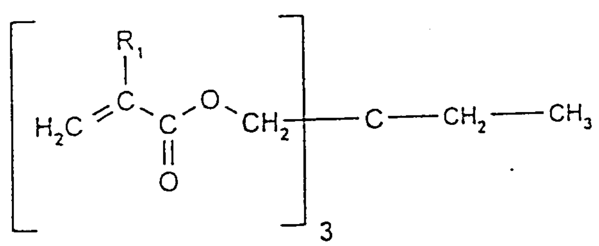

(2),

in der $R_1$ Wasserstoff oder ein $C_{1-4}$-Alkyl bedeutet.

7. Zusammensetzung nach einem der Ansprüche 1 bis 6, dadurch gekennzeichnet, daß die Poly(2-acrylamido-2-methylpropansulfonsäure) mit Trimethylolpropantriacrylat vernetzt ist.

8. Zusammensetzung nach einem der Ansprüche 1 bis 7, dadurch gekennzeichnet, daß die Polymere der Formel (1) eine mit einem BROOKFIELD-Viskosimeter, Drehkörper 4, Geschwindigkeit 100 U/min bei 25 °C in 2%iger wäßriger Lösung gemessene Viskosität größer als oder gleich 1000 mPas (1000 cP) aufweisen.

9. Zusammensetzung nach einem der Ansprüche 1 bis 8, dadurch gekennzeichnet, daß die Polymere der Formel (1) eine mit einem BROOKFIELD-Viskosimeter, Drehkörper 4, Geschwindigkeit 100 U/min bei 25 °C in 2%iger wäßriger Lösung gemessene Viskosität von 5000 bis 40000 mPas (5000 bis 40000 cP) und insbesondere 6500 bis 35000 mPas (6500 bis 35000 cP) aufweisen.

10. Zusammensetzung nach einem der Ansprüche 1 bis 9, dadurch gekennzeichnet, daß die vernetzte Poly(2-acrylamido-2-methylpropansulfonsäure) in einer Konzentration von vorzugsweise 0,1 bis 10 Gew.-%, bezogen auf das Gesamtgewicht der Zusammensetzung, und noch bevorzugter 0,1 bis 0,5 Gew.-% enthalten ist.

11. Zusammensetzung nach einem der Ansprüche 1 bis 10, dadurch gekennzeichnet, daß die mittlere Größe der flüssigen Partikel (oder Kügelchen) der Fettphase in der dispergierenden wäßrigen Phase 70 bis 350 nm und vorzugsweise 70 bis 300 nm beträgt.

12. Zusammensetzung nach einem der Ansprüche 1 bis 11, dadurch gekennzeichnet, daß 90 % der Partikel der ultrafeinen Öl-in-Wasser-Emulsion eine Größe von 100 bis 300 nm haben und daß der Größenunterschied zwischen den größten und den kleinsten Partikeln 20 bis 400 nm und vorzugsweise 30 bis 200 nm beträgt.

13. Zusammensetzung nach einem der Ansprüche 1 bis 12, dadurch gekennzeichnet, daß die dispergierende wäßrige Phase aus Wasser oder einem Gemisch aus Wasser und einem oder mehreren mehrwertigen Alkoholen oder einem Gemisch aus Wasser und einem oder mehreren wasserlöslichen niederen Alkoholen besteht.

14. Zusammensetzung nach einem der Ansprüche 1 bis 13, dadurch gekennzeichnet, daß die dispergierende wäßrige Phase 50 bis 95 Gew.-% und bevorzugter 70 bis 90 Gew.-% der Gesamtmenge der Zusammensetzung ausmacht.

15. Zusammensetzung nach einem der Ansprüche 1 bis 14, dadurch gekennzeichnet, daß die Fettphase aus einer Verbindung oder einem Gemisch von Verbindungen besteht, die unter den verschiedenen Fettsubstanzen, Ölen pflanzlicher, tierischer, synthetischer oder anorganischer Herkunft, natürlichen oder synthetischen Wachsen und dergleichen ausgewählt werden, die üblicherweise in Öl-in-Wasser-Emulsionen verwendet werden.

16. Zusammensetzung nach einem der Ansprüche 1 bis 15, dadurch gekennzeichnet, daß die dispergierte Fettphase 0,5 bis 50 Gew.-%, noch bevorzugter 10 bis 30 Gew.-%, der Gesamtmenge der Zusammensetzung ausmacht.

17. Zusammensetzung nach einem der Ansprüche 1 bis 16, dadurch gekennzeichnet, daß sie mindestens einen Emulgator enthält, der unter nichtionischen Verbindungen ausgewählt ist, die zu einem aus einem lipophilen Rest, der unter $C_{6-30}$-Alkyl- und $C_{6-30}$-Acylgruppen ausgewählt ist, und zum anderen aus einem hydrophilen Rest besteht, der unter Glykol-, Glucoseguppen und den Ethern mehrwertiger Alkohole ausgewählt ist.

18. Zusammensetzung nach Anspruch 17, dadurch gekennzeichnet, daß der Emulgator einen HLB-Wert (hydrophile-lipophile-balance) von 9 bis 18 aufweist.

19. Zusammensetzung nach Anspruch 17 oder 18, dadurch gekennzeichnet, daß der Emulgator unter den Additionsprodukte der Addition von Ethylenoxid an Fettalkohole mit 6 bis 30 Kohlenstoffatomen oder an partielle Ester aus mehrwertigen Alkoholen mit 3 bis 16 Kohlenstoffatomen und Fettsäuren mit 14 bis 22 Kohlenstoffatomen ausgewählt ist.

20. Zusammensetzung nach Anspruch 19, dadurch gekennzeichnet, daß der Emulgator ausgewählt ist unter:

- Verbindungen der Formel (IV)

$$R^4\text{-}(O\text{-}CH_2\text{-}CH_2)_n\text{-}OH \qquad (IV),$$

  in der $R_4$ einen gesättigten oder ungesättigten, verzweigten oder linearen Kohlenwasserstoffrest mit 8 bis 28 Kohlenstoffatomen darstellt und n eine Zahl von 8 bis 50, vorzugsweise 8 bis 30, bedeutet, und
- den Produkten der Addition von 4 bis 20 mol Ethylenoxid an einen oder mehrere partielle Glycerinester.

21. Zusammensetzung nach einem der Ansprüche 17 bis 20, dadurch gekennzeichnet, daß sie außerdem mindestens einen Coemulgator enthält, der unter $C_{16-22}$-Fettalkoholen und den partiellen Estern aus mehrwertigen $C_{3-6}$-Alkoholen und $C_{14-22}$-Fettsäuren ausgewählt ist.

22. Zusammensetzung nach Anspruch 21, dadurch gekennzeichnet, daß sie den oder die (Co)emulgator(en) in einem Anteil von 0,5 bis 30 Gew.-%, noch bevorzugter 2 bis 10 Gew.-%, bezogen auf das Gesamtgewicht der Zusammensetzung, enthält.

23. Zusammensetzung nach einem der Ansprüche 1 bis 22, dadurch gekennzeichnet, daß sie außerdem mindestens einen Zusatz enthält, der ausgewählt ist unter herkömmlichen wäßrigen oder lipophilen Gelbildnern und/oder Verdickungsmitteln, hydrophilen und lipophilen Wirkstoffen, Konservierungsmitteln, Antioxidantien, Parfums, Hydratisierungsmitteln, Weichmachern, Maskierungsmitteln, grenzflächenaktiven Stoffen, Polymeren, Alkalisierungsmitteln und Säuerungsmitteln, Füllstoffen, Mitteln gegen freie Radikale, Ceramiden, Lichtschutzfiltern, Repellentien gegen Insekten, schlanker machenden Mitteln, Farbmitteln, Bakteriziden, Antischuppenmitteln.

24. Nicht-therapeutische Verwendung der Zusammensetzung nach einem der Ansprüche 1 bis 23 als Haarbehandlungsmittel.

25. Nicht-therapeutische Verwendung der Zusammensetzung nach einem der Ansprüche 1 bis 23 als Pflegeprodukt für die Haut, die Haare, die Kopfhaut, die Wimpern, die Augenbrauen, die Nägel oder die Schleimhäute.

26. Nicht-therapeutische Verwendung der Zusammensetzung nach einem der Ansprüche 1 bis 23 als Schminkprodukt.

27. Nicht-therapeutische Verwendung der Zusammensetzung nach einem der Ansprüche 1 bis 23 als Lichtschutzprodukt.

28. Verfahren zur nicht-therapeutischen kosmetischen Behandlung der Haut, der Kopfhaut, der Haare, der Wimpern, der Augenbrauen, der Nägel oder der Schleimhäute, dadurch gekennzeichnet, daß auf den Träger eine wie in einem der Ansprüche 1 bis 23 definierte Zusammensetzung aufgetragen wird.

29. Verfahren zur Herstellung einer Zusammensetzung nach einem der Ansprüche 1 bis 23, dadurch gekennzeichnet, daß sie dadurch erhalten wird, daß

(A) mindestens ein kosmetisches Öl,
(B) mindestens ein Emulgator und gegebenenfalls ein Coemulgator,
(D) Wasser
und gegebenenfalls
(E) bei der Phaseninversionstemperatur stabile Hilfs stoffe,

vermischt werden, um eine herkömmliche Emulsion zu erhalten, daß dann diese Emulsion auf eine Temperatur innerhalb oder oberhalb des Phaseninversionsbereichs erwärmt wird oder daß die Emulsion bei einer derartigen Temperatur hergestellt wird, daß dann in einem zweiten Schritt die Emulsion auf eine Temperatur unterhalb des Phaseninversionsbereichs abgekühlt wird, wonach die erhaltene Emulsion in ein Gel übergeführt wird, indem (C) ein vernetztes und fast vollständig oder vollständig neutralisiertes, wie weiter oben beschriebenes Poly(2-acrylamido-2-methylpropansulfonsäure)-Polymer zugegeben wird.

30. Verwendung der Zusammensetzung nach einem der Ansprüche 1 bis 23 für die Herstellung einer Pomade oder einer Salbe, die für die therapeutische Behandlung des Gesichts und/oder der Hände und/oder des menschlichen Körpers vorgesehen ist.

31. Verwendung eines wie in einem der Ansprüche 1 bis 9 beschriebenen Polymers für die Überführung in ein Gel und/oder die Verdickung einer kosmetischen und/oder dermatologischen Zusammensetzung, die in Form einer ultrafeinen Öl-in-Wasser-Emulsion vorliegt, in der die mittlere Größe der Kügelchen, die die Ölphase bilden, 50 bis 1000 nm beträgt.

32. Verwendung eines wie in einem der Ansprüche 1 bis 9 definierten Polymers für die Stabilisierung einer kosmetischen und/oder dermatologischen Zusammensetzung, die in Form einer ultrafeinen Öl-in-Wasser-Emulsion vorliegt, in der die mittlere Größe der Kügelchen, die die Ölphase bilden, 50 bis 1000 nm beträgt.